# EUROPÄISCHE PATENTSCHRIFT

(11) **EP 2 113 284 B1**
(45) Veröffentlichungstag und Bekanntmachung des Hinweises auf die Patenterteilung: **05.06.2013**
(21) Anmeldenummer: 09003976.9
(22) Anmeldetag: 19.03.2009
(51) Int. Cl.: A61N 2/02, A61B 17/86, H01F 5/00

(54) **Vorrichtung zum Stimulieren eines Heilungsprozesses**
Device for stimulating a healing process
Dispositif de stimulation d'un processus de guérison

(30) Priorität: 30.04.2008 DE 102008021575
(43) Veröffentlichungstag der Anmeldung: 04.11.2009
(73) Patentinhaber: Neue Magnetodyn GmbH, 80333 München (DE)
(72) Erfinder: Kraus, Werner, 80539 München (DE); Kraus, Stephanie, 80538 München (DE); Stephan, Heribert, 80689 München (DE)
(74) Vertreter: Schumacher & Willsau

(56) Entgegenhaltungen:
- EP-A2- 2 074 958
- WO-A1-2008/035089
- WO-A2-2005/074821
- WO-A2-2007/124731
- US-A- 4 501 265

## Beschreibung

Die Erfindung betrifft eine Vorrichtung zum Stimulieren eines Heilungsprozesses.

Der Einsatz elektromagnetischer Felder bei der Behandlung ausgedehnter Wunden des Weichgewebes, der Muskulatur und der Haut kann häufig den Heilungsprozess deutlich beschleunigen. Insbesondere existieren Erkrankungen, die im Hinblick auf eine antibiotische Behandlung therapieresistent sind, wobei letztere beispielsweise durch MRSA (Methicillin-resistenter Staphylococcus aureus), und andere Keime wie Enterokokken oder Pseudomonaden verursacht werden können. Diese Therapieresistenz kann durch die Anwendung elektromagnetischer Felder überwunden werden.

Therapieerfolge sind beispielsweise bei der Behandlung von schlecht heilenden Knochenfrakturen sowie damit in Verbindung stehender meist bakteriell verursachter Sekundärerkrankungen zu verzeichnen. Auch das so genannte "offene Bein" (Ulcus cruris) lässt sich mit elektromagnetischen Feldern erfolgreich therapieren. Weiterhin spielen dermatologische Anwendungen, beispielsweise zur Behandlung von großflächigen chronischen Wunden der Haut, wie Ulcera cruris oder Verbrennungen, im Zusammenhang mit der Therapie mittels elektromagnetischer Felder eine wichtige Rolle. Zum Beispiel können Verbrennungen höheren Grades Hauttransplantationen erforderlich machen, die durch von außen angewendete Magnetfelder vorbereitet und nach der Transplantation beim Einheilen unterstützt werden können.

Die applizierten elektromagnetischen Wechselfelder haben beispielsweise eine Magnetfeldstärke von 1 bis 5 mT und einen sinusförmigen Verlauf mit einer Frequenz von 1 bis 20 Hz. Die Erzeugung der Felder erfolgt durch Magnetspulen, die mit einem entsprechenden Wechselstrom erregt werden. Dabei können verschiedene Spulenanordnungen zum Einsatz kommen, beispielsweise Solenoidspulen oder Helmholtzspulen, je nach Anwendung und gewünschter Magnetfeldrichtung.

In zahlreichen Untersuchungen der Grundlagenforschung in vivo und in vitro zeigte sich eine spezifische Zuordnung von Wachstum und Differenzierung humaner Bindegewebe und Knochenzellen, nämlich Fibroblasten und Osteoblasten, zu den Einflussparametern des elektrischen und des magnetischen Feldes. Proliferierende Zellen und Gewebewachstum wurden überwiegend im Bereich der elektrischen Feldgradienten, das heißt insbesondere in der Umgebung von Elektroden, die im galvanischen Kontakt mit dem Gewebe stehen, beobachtet. Hingegen wuchs die Zahl differenzierender Zellen, mit einer entsprechenden Steigerung ihres Stoffwechsels und der kontrollierten Synthese von Strukturproteinen - bei abnehmenden Mitoseraten - im ausgedehnten Volumen des magnetfelddurchfluteten Gewebes.

Die WO 2007/124731 lehrt eine Vorrichtung gemäß dem Oberbegriff des Anspruchs 1.

Der Erfindung liegt die Aufgabe zugrunde, eine auf der Grundlage elektromagnetischer Wechselfelder arbeitende Stimulationsvorrichtung zu schaffen, die geeignet ist, die Heilung eines erkrankten Körperbereiches in den verschiedenen Heilungsphasen angepasster Weise zu unterstützen.

Diese Aufgabe wird mit den Merkmalen des unabhängigen Anspruchs gelöst.

Vorteilhafte Ausführungsformen der Erfindung sind in den abhängigen Ansprüchen angegeben.

Die Erfindung besteht in einer Vorrichtung zum Stimulieren eines Heilungsprozesses mit einer mit einem Funktionsstromgenerator gekoppelten Spulenanordnung zum Erzeugen eines elektromagnetischen Feldes in einem erkrankten Körperbereich, einer Steuereinheit zum Beeinflussen eines von dem Funktionsstromgenerator erzeugten Spannungsverlaufs in Abhängigkeit von an eine Eingangsschnittstelle der Steuereinheit übertragenen Signalen, einer Sensoranordnung zum Erfassen einer Eigenschaft des erkrankten Körperbereichs und einer mit der Sensoranordnung gekoppelten Übertragungseinrichtung zum Übertragen von für die erfasste Eigenschaft des erkrankten Körperbereichs charakteristischen Signalen an die Steuereinheit. Die Stimulationsvorrichtung kann somit das elektromagnetische Feld in dem erkrankten Körperbereich in Abhängigkeit der von der Sensoranordnung erfassten Eigenschaften modulieren. Wenn sich die von der Sensoranordnung erfassten Eigenschaften des erkrankten Körperbereiches im Verlaufe der Heilung verändern, kann aufgrund der Modulationsfähigkeit stets eine in optimaler Weise angepasste Unterstützung des Heilungsprozesses erfolgen, was letztlich zu einer Beschleunigung des Heilprozesses sowie einem verbesserten Therapieergebnis führt.

In diesem Zusammenhang ist es von besonderem Vorteil, dass die Sensoranordnung einen pH-Sensor umfasst. Der im Bereich erkrankter Körperregionen vorliegende pH-Wert ist ein Indiz für die biochemische Qualität des Gewebes. Zum Beispiel kann im Bereich einer großflächigen Weichteil-Verletzung, bei Verbrennungen zweiten bis dritten Grades oder bei pathologisch veränderten ulcerierenden Wunden die Wirksamkeit therapeutischer Maßnahmen, wie Wundverbände oder Hauttransplantationen einschließlich chemischer Antibiosen bei bakteriellen Infektionen, an der Änderung des Wund-pH-Wertes ermessen und eine entsprechende Änderung der Behandlungsmethodik vorgenommen werden. Den Anforderungen einer therapeutisch optimalen Regulation des Wundmilieus wird mit einer pH-Wert-kontrollierten und -modifizierten Induktion des lädierten Weichgewebes durch extrem niederfrequente, nicht-thermische magnetische und elektrische Felder mit zeitlich veränderbaren Verlaufs-(Signal)-Formen entsprochen. Anstelle oder neben pH-Sensoren können auch andere Sensoren zum Einsatz kommen, nämlich beispielsweise Temperatursensoren, Leitfähigkeitssensoren und/oder Sensoren zum Erfassen der Anwesenheit und/oder der Konzentration bestimmter chemischer Substanzen.

Gemäß einer Ausführungsform der vorliegenden Erfindung ist vorgesehen, dass die Übertragungseinrichtung mindestens eine elektrische Leitung umfasst, die direkt mit der Eingangsschnittstelle der Steuereinheit in Verbindung steht. Die von der Sensoranordnung gelieferten elektrischen Signale können auf diese Weise an die Steuereinheit des Funktionsstromgenerators ohne besonderen technischen Aufwand übertragen werden.

Es ist aber auch möglich, dass die Übertragungseinrichtung mindestens einen Sender zur drahtlosen Kommunikation mit einem der Eingangsschnittstelle der Steuereinheit zugehörigen Empfänger aufweist. Ein solcher Sender kann in verschiedener Weise ausgestaltet sein. Wesentlich ist, dass er die von der Sensoranordnung erfassten Messwerte in der Weise umsetzt, dass entsprechende Signale an den Empfänger der Steuereinheit übermittelt werden können. Eine Möglichkeit einer solchen Informationsübertragung vom Sender zum Empfänger in der Steuereinheit besteht in der aktiver Erzeugung von Sendesignalen, die von den Messwerten der Sensoranordnung abhängen.

Es kann aber auch vorgesehen sein, dass die Übertragungseinrichtung mindestens einen RFID-Transponder aufweist, dessen Informationsgehalt von einem der Eingangsschnittstelle der Steuereinheit zugehörigen Lesegerät erfassbar ist. Bei einem RFID-Transponder handelt es sich um eine Einrichtung, die nur durch ihr Zusammenspiel mit einem Lesegerät Informationen "aussenden" kann. Letztlich empfängt der RFID-Transponder zu diesem Zweck ein elektromagnetisches Hochfrequenzfeld, das von dem Lesegerät erzeugt wird, um dieses dann in Abhängigkeit von in dem RFID-Transponder gespeicherten Informationen zu verändern. Diese Veränderung wird vom Lesegerät erfasst. Aufgrund dieser im Vergleich zu herkömmlichen aktiven Sendern sehr eingeschränkten Funktionalität eines RFID-Transponders, ist dieser kostengünstig und platzsparend.

Die Informationsübertragung von dem RFID-Transponder zu dem Lesegerät kann auf der Grundlage stattfinden, dass der auslesbare Informationsgehalt des RFID-Transponders in Abhängigkeit von Signalen, die von der Sensoranordnung geliefert werden, veränderbar ist. Im einfachsten Fall werden von der Sensoranordnung verschiedene Spannungen an den Speicher des RFID-Transponders angelegt, wobei diese Spannungen die durch die Sensoranordnung erfassten Eigenschaften widerspiegeln. Verschiedene Spannungen können nun bewirken, dass der Speicherinhalt des RFID-Transponders verändert wird, so dass letztlich auch die von dem RFID-Transponder an das Lesegerät übertragene Kennung geändert wird. Damit die Möglichkeit besteht, den Speicherinhalt des RFID-Transponders zu verändern, ist es erforderlich, beschreibbare RFID-Transponder zu verwenden.

Alternativ oder zusätzlich ist es aber auch möglich, dass mehrere RFID-Transponder vorgesehen sind, die in Abhängigkeit von Signalen, die von der Sensoranordnung geliefert werden, aktivierbar oder deaktivierbar sind. In diesem Fall sind nicht beschreibbare Transponder ausreichend. Eine oder mehrere Schwellenwertschaltungen, in die die Sensoranordnung und die RFID-Transponder eingebunden sind, sorgen dafür, dass in Abhängigkeit der von der Sensoranordnung gelieferten Spannungen unterschiedliche RFID-Transponder aktiv beziehungsweise inaktiv sind. Auch auf diese Weise kann das Lesegerät also unterschiedliche Kennungen je nach der von der Sensoranordnung gelieferten Spannung empfangen, also auch auf dieser Grundlage dafür sorgen, dass der Funktionsstromgenerator einen an die erfassten Eigenschaften des erkrankten Körperbereiches angepassten Spannungsverlauf erzeugt.

Nützlicherweise ist vorgesehen, dass die Sensoranordnung einen ionensensitiven Feldeffektransistor aufweist. Diese Halbleiterbauelemente eignen sich zur Erfassung des pH-Wertes, und sie können in sehr kleiner Bauart hergestellt und erworben werden, so dass sie im Zusammenhang mit zahlreichen Erkrankungen eingesetzt werden können.

Die Erfindung ist in besonders vorteilhafter Weise dadurch weitergebildet, dass die Spulenanordnung eine Spule mit einer einen Kreuzungspunkt aufweisenden Spulenwicklung aufweist, die durch eine achtförmige Gestalt zwei Flächen definiert, wobei die Flächen so zueinander ausgerichtet sind, dass die von einem durch einen Stromfluss in der Spulenanordnung erzeugten, die Flächen durchdringenden Magnetfelder im Wesentlichen gleichgerichtet sind. Die Wirkung von zwei getrennten Induktionsspulen mit gleicher Richtung ihres Magnetfeldes, in dem die lädierte Körperregion gelagert wird, kann auf diese Weise auch mit einer einzigen Spule erreicht werden. Dies macht die Anwendung komfortabel, insbesondere aufgrund des verminderten apparativen Aufwands.

Nützlicherweise ist vorgesehen, dass die Spulenanordnung flexibel ist, so dass die Flächen auf gegenüberliegenden Seiten einer einem Magnetfeld auszusetzenden Körperregion angeordnet werden können. Durch ihre flexible Wicklung, die insbesondere durch eine Elastizität der beteiligten Materialien bewirkt sein kann, wird die Umformung im Sinne eines Achter- oder Unendlich-Zeichens ermöglicht. Die sich durch diese Umformung der Spulenwicklung ergebenden "Schlaufen" der Spule können beispielsweise an beiden Seiten einer Extremität angelegt werden. Sie können je nach Anwendung gleiche oder unterschiedliche Größen haben. Die Spulenanordnung kann durch Ihre Flexibilität sehr vielseitig eingesetzt werden. Im Zusammenhang mit der Auslegung der Spulenanordnung kann vorgesehen sein, dass an zwei dem Kreuzungspunkt abgewandten Positionen der Spulenanordnung zusammenwirkende Befestigungsmittel vorgesehen sind, durch die die Ausrichtung der Flächen zueinander geschaffen und aufrechterhalten werden kann. Die Befestigungsmittel können beispielsweise mittels Gurten, Druckknöpfen, Klettverschlüssen, Schnallen und dergleichen realisiert sein. In besonders vorteilhafter Weise kann vorgesehen sein, dass der Kreuzungspunkt der Spulenanordnung durch eine Kopplungseinrichtung fixierbar ist. Eine solche Kopplungseinrichtung kann beispielsweise durch ein elastisches oder flexibles Band mit Klettverschluss oder Gürtelschließe realisiert sein. Es ist von besonderem Vorteil, dass die Lage des Kreuzungspunktes und somit die Maße der Flächen variabel sind. Durch das auf diese Weise wählbare Flächenverhältnis der Spulenschlaufen ist die magnetische Induktionsflussdichte, die als Quotient aus dem magnetischen Fluss und der betrachteten Fläche definiert ist, einstellbar. Das Verhältnis der Induktionsflussdichten ist der Kehrwert des Verhältnisses der jeweils zugehörigen Flächen. Das Flächenverhältnis der beiden Schlaufen kann nach den therapeutischen Erfordernissen gewählt werden, indem eine veränderbare Fixierung des Kreuzungspunktes durch variable Anordnung der Kopplungseinrichtung ermöglicht wird. Beispielsweise kann in einem Zielbereich des Körpers eine hohe magnetische Induktionsflussdichte dadurch erzielt werden, dass eine kleinflächige Spulenschlaufe in dessen Nähe gebracht wird, während im Hinblick auf die andere großflächige Spulenfläche zur Bereitstellung des Helmholtzspuleneffektes hauptsächlich auf deren parallele Anordnung zur kleineren Fläche zu achten ist. Mit einer solchen Spulenanordnung wird erreicht, dass das Magnetfeld der beiden sich gegenüberstehenden Schlaufen der Spulenanordnung durch die räumliche Umkehr der Stromrichtung in einer der beiden Schlaufen, wie bei der Anordnung von zwei getrennten Spulen nach Helmholtz, gleichgerichtet ist, wobei eine besonders gute und flexible Handhabbarkeit zur Verfügung gestellt wird. Eine einfache Handhabung bei der Anpassung der geometrischen Form der Spulenanordnung an die Lage der jeweiligen Knochen bzw. Weichgewebeläsion wird ermöglicht. Beispielsweise können die Schlaufenflächen an Behandlungsbereiche wie Fuß, Knie Unterschenkel, Oberschenkel, Becken, Wirbelsäule, Hand, Unterarm, Oberarm, Kiefer und Schädelbereich angepasst werden; durch Variation der Schlaufengrößen auch im Hinblick auf die Stärke des Magnetfeldes. Eine weitere Eigenschaft der erfindungsgemäßen Spulenanordnung ist eine Zunahme der Flussdichte im Nahbereich des Kreuzungspunktes, so dass eine relativ starke Magnetfeldkonzentration auf eine kleine Körperfläche ermöglicht wird. Auf diese Weise lassen sich stark lokalisierte Erkrankungen, wie zum Beispiel Abszesse und Infekte, wirkungsvoll behandeln. Gemäß einer besonders bevorzugten Ausführungsform der Erfindung ist die Spulenanordnung derart weitergebildet, dass sie plastische Eigenschaften hat. Durch die Flexibilität der Spule wird eine Anformung an die zu therapierende Körperregion ermöglicht, während die Plastizität die Spulenanordnung in der angeformten Position hält. Die plastischen Eigenschaften können somit unterstützend zu sonstigen Befestigungsvorrichtungen, wie etwa Schnallen oder Klemmen, wirken oder auch allein für den Halt der Spule in der gewünschten Form sorgen. Nützlicherweise ist vorgesehen, dass die plastischen Eigenschaften durch mindestens eine plastische Materialkomponente vermittelt werden, die mindestens eine nicht plastische Materialkomponente umgibt und/oder von dieser eingebettet ist. Die Spule kann also einerseits aus einem flexiblen Material gefertigt werden, ohne dass es darauf ankäme, ob dieses plastische Eigenschaften hat. Die plastischen Eigenschaften werden der Spule dann durch eine plastische Materialkomponente vermittelt, so dass die Spule insgesamt in der durch die Verformung geschaffenen Stellung verbleibt. Dies kann insbesondere dadurch realisiert sein, dass die mindestens eine plastische Materialkomponente durch mindestens eine sich im Wesentlichen parallel zu den Spulenwicklungen erstreckende Ader aus plastischem Material gebildet ist. Eine oder mehrere solche Adern verlaufen ganz oder teilweise parallel zu den Wicklungen der Spule. Eine in dieser Art und Weise aufgebaute Spule ist besonders einfach zu fertigen.

Die Erfindung ist in besonders vorteilhafter Weise dadurch weitergebildet, dass der Funktionsstromgenerator geeignet ist, in Abhängigkeit von an die Eingangsschnittstelle der Steuereinheit übertragenen Signalen quasi rein-harmonische Spannungsverläufe mit einem ersten Oberwellenanteil oder entartet-harmonische Spannungsverläufe mit einem zweiten Oberwellenanteil, der größer als der erste Oberwellenanteil ist, zu erzeugen. Durch die Veränderung des von dem Funktionsstromgenerator erzeugten Spannungsverlaufs können die Oberwellenanteile des elektromagnetischen Feldes im erkrankten Körperbereich verändert werden. Insbesondere kann dabei die Frequenz des niederfrequenten Magnetfeldes aufrechterhalten werden, während aber elektrische Felder, welche vom zeitlichen Differential des Magnetfeldes abhängen, bis hin zu hohen Frequenzen variiert werden können. Man kann auf diese Weise den erkrankten Körperbereich also quasi unveränderlichen niederfrequenten magnetischen Wechselfeldern aussetzen, die die Differenzierung der Zellen begünstigen, während hochfrequente Anteile dann erzeugt werden, wenn dies aufgrund der von der Sensoranordnung erfassten Werte, insbesondere pH-Werte, sinnvoll ist.

Konkret ist im Falle der Wundheilung am menschlichen Körper besonders bevorzugt, dass der Funktionsstromgenerator geeignet ist, bei einem von der Sensoranordnung erfassten pH-Wert unter 7 einen quasi rein-harmonischen Spannungsverlauf mit einer Frequenz zwischen 1 und 30 Hz zu erzeugen und bei einem von der Sensoranordnung erfassten pH-Wert über 7 einen entartet-harmonischen Spannungsverlauf mit einer Grundfrequenz zwischen 1 und 30 Hz und physiologisch wirksamen Oberwellen mit einem fünf- bis fünfzigfachen der Grundfrequenz zu erzeugen. Während niedrige pH-Werte unter 7 bei Behandlungsbeginn und Behandlungsende vorliegen, beobachtet man in der Hauptphase des Heilprozesses, nämlich in der Wachstumsphase des Bindegewebes, pH-Werte oberhalb von 7. Während dieser Wachstumsphase ist es von besonderem Vorteil, den Wundbereich einer hohen elektrischen Feldstärke auszusetzen, während dies am Anfang des Prozesses nicht erforderlich und insbesondere am Ende der Behandlung, wenn der pH-Wert wieder auf Werte unterhalb von 7 absinkt, er kontraindiziert ist, so dass die elektrische Stimulation des Gewebewachstums zugunsten der magnetisch stimulierten Differenzierung von Zellen und Gewebestrukturen wieder zurückgeführt werden kann. Der Begriff "physiologisch wirksame Oberwellen" bedeutet im vorliegenden Zusammenhang, dass diese eine Intensität aufweisen, die den Heilungsverlauf mit noch nachweisbarer Auswirkung beeinflussen. Beispielsweise kann die Intensität solcher noch physiologisch wirksamer Oberwellen im Bereich zwischen 5 und 15 % der Intensität der Grundschwingung liegen.

Gemäß einer weiteren bevorzugten Ausführungsform der Erfindung ist vorgesehen, dass mit einer Übertragerspule gekoppelte Elektroden zur Anordnung in oder an dem erkrankten Körperbereich vorgesehen sind. Derartige Elektroden, die insbesondere auch als Wundelektroden bezeichnet werden, können speziell bei therapieresistenten Erkrankungen zum Einsatz kommen. Zusätzlich zu der ohnehin vorhandenen Felddurchdringung des erkrankten Körperbereiches wird auch eine Übertragerspule von dem externen Magnetfeld angeregt, so dass aufgrund der Kopplung der Elektroden mit der Übertragerspule ein elektrisches Wechselfeld gezielt und verstärkt in den Wundbereich eingebracht werden kann.

Die vorliegende Erfindung betrifft eine Vorrichtung zum Stimulieren eines Heilungsprozesses. Die Erfindung ließe sich aber auch als Verfahren formulieren, bei dem es wesentlich auf die folgenden Verfahrensschritte ankommt: Erzeugen eines elektromagnetischen Feldes in einem erkrankten Körperbereich; Erfassen einer Eigenschaft des erkrankten Körperbereichs; Übertragen von für die erfasste Eigenschaft des erkrankten Körperbereichs charakteristischen Signalen an eine Steuereinheit eines Funktionsstromgenerators; Beeinflussen eines von dem Funktionsstromgenerator erzeugten Spannungsverlaufs in Abhängigkeit der übertragenen Signale. Besonders vorteilhafte Ausführungsformen dieses Verfahrens sind **dadurch gekennzeichnet, dass** ein pH-Wert als Eigenschaft des erkrankten Körperbereiches gemessen wird und/oder die Übertragung der Informationen an die Steuereinheit unter Verwendung von mindestens einem RFID-Transponder erfolgt. Weiterhin ist das Verfahren besonders nützlich in der Weise ausgebildet, dass bei einem erfassten pH-Wert unter 7 ein quasi rein-harmonischer Spannungsverlauf mit einer Frequenz zwischen 1 und 30 Hz erzeugt wird und bei einem pH-Wert über 7 ein entartet-harmonischer Spannungsverlauf mit einer Grundfrequenz zwischen 1 und 30 Hz und physiologisch wirksamen Oberwellen mit einem fünfbis fünfzigfachen der Grundfrequenz erzeugt wird.

Die Erfindung wird nun mit Bezug auf die begleitenden Zeichnungen anhand besonders bevorzugter Ausführungsformen beispielhaft erläutert.

### Es zeigen:

- Figur 1: eine erfindungsgemäße Vorrichtung während ihrer Anwendung;
- Figur 2: einen typischen Verlauf des pH-Wertes in einer großflächigen sekundärheilenden Wunde über 30 Tage;
- Figur 3: einen rein-harmonischen Spannungsverlauf;
- Figur 4: einen entartet-harmonischen Spannungsverlauf;
- Figur 5: eine in einer erfindungsgemäßen Vorrichtung einsetzbare Spulenanordnung in einem ersten Zustand;
- Figur 6: eine in einer erfindungsgemäßen Vorrichtung einsetzbare Spulenanordnung in einem zweiten Zustand;
- Figur 7: eine schematische Darstellung zur Erläuterung der räumlichen Ausrichtung eines durch eine erfindungsgemäße Spulenanordnung erzeugten Magnetfeldes;
- Figur 8: eine geschnittene perspektivische Teildarstellung einer Spulenanordnung mit plastischen Eigenschaften und
- Figur 9: eine Anordnung, die im Rahmen einer erfindungsgemäßen Vorrichtung einsetzbar ist.

Bei der nachfolgenden Beschreibung der Zeichnungen bezeichnen gleiche Bezugszeichen gleiche oder vergleichbare Komponenten.

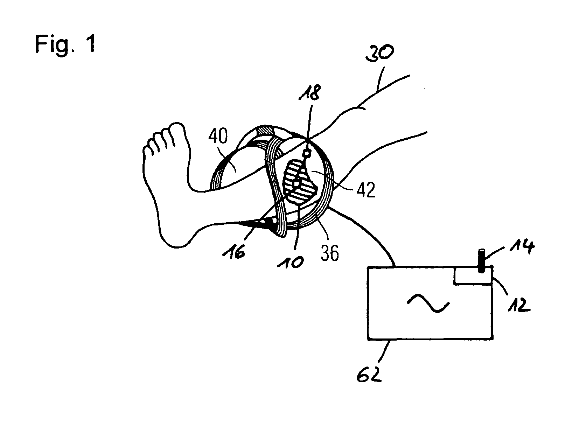

Figur 1 zeigt erfindungsgemäße Vorrichtung während ihrer Anwendung. Es ist ein Bein 30 mit einem Wundbereich 10 am Unterschenkel zu erkennen. Das induzierende elektromagnetische Feld wird in einer flexiblen Magnetspule 36 von einem Funktionsstromgenerator 62 erzeugt. Zur besseren Darstellung wurde die flexible Spule 36 mit einem Abstand zum erkrankten Körperbereich dargestellt. In der Praxis bei der Behandlung einer großflächigen Wunde 10 wird die Magnetspule 36 jedoch vorzugsweise unmittelbar an die lädierte Körperregion angeformt, so dass ihre Wicklung den Wundbereich 10 in geringem Abstand umfängt. Die Vektoren des Spulenfeldes durchdringen die Wundfläche 10 ungefähr im rechten Winkel. Für die Messung des Wund-pH-Wertes wird mindestens ein pH-Sensor 16 in Form eines ionensensitiven Feldeffekttransistors in direktem Kontakt mit dem lädierten Gewebe durch einen Verband (nicht dargestellt) oder ein Klebepflaster (nicht dargestellt) an der gewünschten Stelle (Wundmitte oder Wundrand) fixiert. Der gemessene pH-Wert wird über Kabel oder unter Vermittlung eines oder mehrerer elektrisch mit dem Sensor 16 verbundenen RFID-Transponder 18 zur Eingangsschnittstelle 14 der Steuereinheit 12 des Funktionsstromgenerators der Magnetspule übertragen, so dass der pH-Wert die Grundlage für die Modulation des die Spule 36 erregenden Spannungsverlaufs und somit des die Wunde 10 durchflutenden Elektromagnetfeldes bilden kann. Um eine störungsfreie Übertragung der hochfrequenten elektromagnetischen Signale zwischen dem RFID-Transponder 18 und der als Lesegerät 14 ausgebildeten Eingangsschnittstelle der Steuereinheit 12 zu gewährleisten, kann es erforderlich sein, die Erzeugung elektromagnetischer Felder in der Magnetspule 36 zum Zwecke der Kommunikation für kurze Zeit auszusetzen.

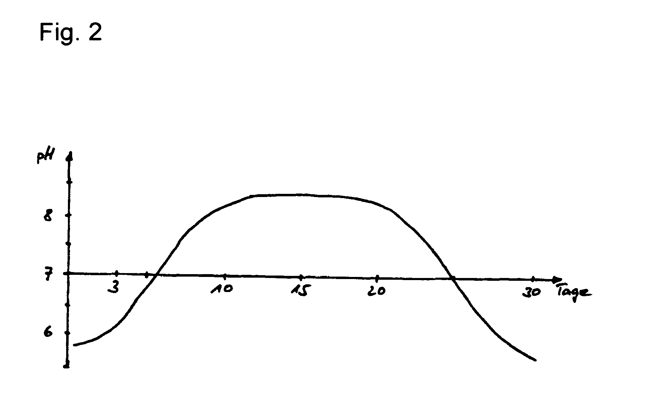

Figur 2 zeigt einen typischen Verlauf des pH-Wertes in einer großflächigen sekundärheilenden Wunde über 30 Tage. Zu Beginn der Behandlung bei einem pH unter 7 erfolgt häufig ein chirurgisches Debridement, das die Wunde von nekrotischem Gewebe befreien und die Einsprossung von Blutgefäßen (Kapillaren) anregen soll. Der pH-Wert steigt bis zum Neutralwert 7. Am dritten bis fünften Tag danach beginnt bereits die Synthese des kollagenen Bindegewebes durch Fibroblasten, die in der unmittelbaren Umgebung der Blutgefäße entstehen. Bei steigendem pH-Wert (pH > 7 bis 8) entwickelt sich im weiteren Verlauf das eigenständige Granulationsgewebe, das auch die biologische und biomechanische Basis für ein eventuell zu applizierendes Hauttransplantat bildet. Die Reepithelisierung, die Endphase der Wundheilung, zeigt sinkende pH-Werte unterhalb von 7, bis hin zu einem pH-Wert von 4, die den schützenden Säuremantel der unverletzten Haut charakterisieren. Einen Haupteinflussfaktor auf den pH-Wert bildet die Sauerstoffatmung der Zelle. Die Aufnahme - paramagnetischer - O₂-Moleküle und ihre Phosphorylierung zu ATP - dem Energiespeicher der Zelle - an den Innenmembranen (Mitochondrien), wird überwiegend vom Magnetfeld induziert, das im Gegensatz zum elektrischen Feld, die Zelle unverändert durchdringt. Durch reduktive und oxidative Reaktionen zur Ladungstrennung am Beginn der Atmungskette werden pro O₂-Molekül zwei Protonen(2H⁺) ausgeschieden. Der extrazellulär sinkende pH-Wert bildet ein neuro-vegetativ wirksames Signal, das eine regulative Steigerung der Durchblutung im Wundbereich verursacht.

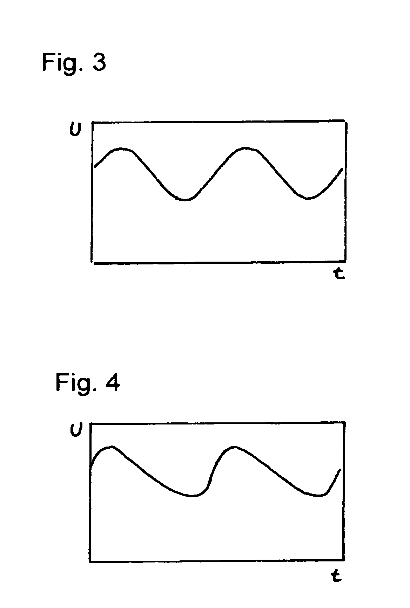

Die Figuren 3 und 4 zeigen Signalverläufe, die von der erfindungsgemäßen Vorrichtung erzeugt werden können. Die Grundform bilden gemäß Figur 3 biphasische Schwingungen der Sinusform im Frequenzbereich von 1 bis 30 Hz und mit einer magnetischen Flussdichte von 0,01 bis 5 mT bei einem Anteil von Oberwellen kleiner oder gleich etwa 1 %. Die induzierte elektrische Komponente (E) erreicht beispielsweise bei einer Sinusschwingung mit einer Frequenz (f) von 20 Hz, im Radius (r) einer Wundfläche von 1 bis 3 cm und einer Flussdicht B von 5 mT (E = π·f·r·B) ca. 3 bis 9 mV/cm. Diese weitgehend oberwellenfreie Grundform des elektromagnetischen Feldes entspricht mit ihrer relativ geringen elektrischen Induktion von weniger als 1 mV/cm einem pH-Wert von unter 7, beispielsweise des sauren Wundsekretes, das unmittelbar nach Behandlungsbeginn, nämlich am ersten bis dritten Tag verstärkt auftritt. Bei einem steigenden pH-Wert von etwa 7 beginnt mit zunehmender Zellproliferation das Wachstum des Bindegewebes. Seine Stimulation wird bei gleichbleibender Frequenz des induzierenden elektromagnetischen Feldes von 20 Hz durch eine dem ansteigenden Verlauf des pH-Werts entsprechende Veränderung des induzierenden elektromagnetischen Feldes erreicht, indem gemäß Figur 4 die Steilheit der ansteigenden Flanken der Sinusschwingung moduliert werden, bis der für die Wachstumsphase charakteristische Wert von pH 8 gemessen werden kann. Ebenfalls ist es möglich die abfallende Flanke der Schwingung mit einem größeren absoluten Steigungswert zu verwehen. Die Änderung der Flankensteilheit dB/dt des induzierenden elektromagnetischen Feldes entspricht maximal der einer physiologisch wirksamen Sinusfrequenz von 1 kHz, vorzugsweise von 200 bis 500 Hz (entsprechend Oberwellen mit einer Frequenz bis zum fünfzigfachen, vorzugsweise dem zehn- bis fünfundzwanzigfachen der Grundschwingungsfrequenz von 20 Hz). Der Wert pH 8 kennzeichnet die Entwicklung des eigenständigen Granulationsgewebes, das den biologischen Boden für das Hauttransplantat bildet. Der das Wachstum stimulierende Schwingungsverlauf des elektromagnetischen Feldes wird beibehalten, bis ein sinkender pH-Wert das Ende der Wachstumsphase und den Beginn der Reepithelisierung anzeigt. Im Verlauf der Hautbildung mit pH-Werten unterhalb 7 oder sogar im Bereich von pH 4 ist die elektrische Stimulation des Gewebewachstums zu Gunsten der magnetisch stimulierten Differenzierung von Zellen und Gewebestrukturen durch die Rückkehr zur zeitlich symmetrischen und im Wesentlichen oberwellenfreien Sinusschwingung zu minimieren. Dadurch wird ein undifferenziert überschießendes Wachstum von Bindegewebe, wie die Bildung von Narben-Keloid, vermieden. Die mit der erfindungsgemäßen Vorrichtung stimulierte Narbe ist von unauffälliger Form und erreicht die elastische Funktion der sie umgebenden unversehrten Haut.

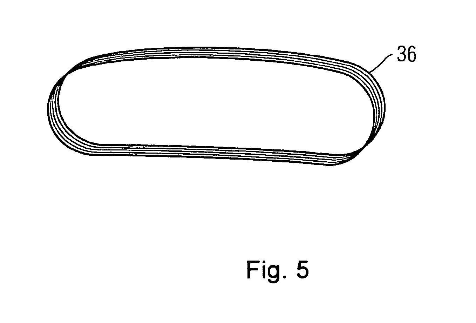

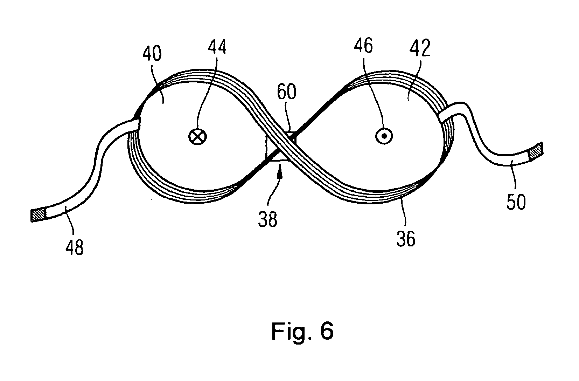

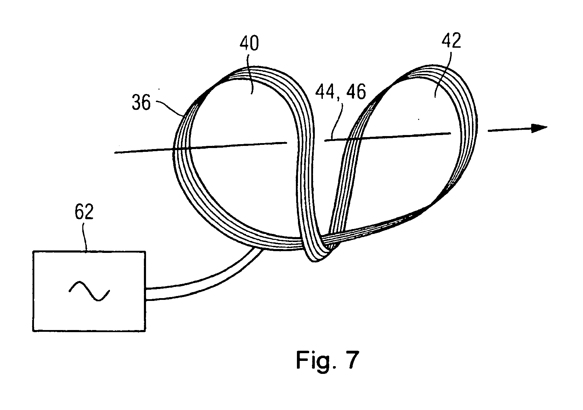

Figur 5 zeigt eine in einer erfindungsgemäßen Vorrichtung einsetzbare Spulenanordnung in einem ersten Zustand. Figur 6 zeigt eine in einer erfindungsgemäßen Vorrichtung einsetzbare Spulenanordnung in einem zweiten Zustand. Figur 7 zeigt eine schematische Darstellung zur Erläuterung der räumlichen Ausrichtung eines durch eine erfindungsgemäße Spulenanordnung erzeugten Magnetfeldes. Nutzt man die Flexibilität der Spulenanordnung 36 dazu aus, diese in eine achtförmige Gestalt zu bringen, so werden zwei Flächen 40, 42 sowie ein Kreuzungspunkt 38 definiert. Der Kreuzungspunkt 38 ist durch eine Kopplungseinrichtung 60 fixiert und kann vorzugsweise verschoben werden, so dass das Verhältnis der Flächen 40, 42 variabel ist. Beispielsweise kann als Kopplungseinrichtung 60 ein elastisches Band mit Klettverschluss oder Gürtelschließe dienen. Ebenfalls ist es denkbar, Oberflächen der Spulenanordnung so auszustatten, dass sie die Komponenten eines Klettverschlusses bilden und auf diese Weise an verschiedenen Stellen direkt aufeinander Haften können. Um die Einstellung zu erleichtern, können im Variationsbereich Vermerke oder Skalen vorgesehen sein; an diesen kann sich der Anwender orientieren, wenn er eine bestimmte Fixierung des Kreuzungspunktes 38 vornehmen möchte. Fließt durch eine solche Spulenanordnung 36 ein Wechselstrom, so haben die induzierten Magnetfelder entgegengesetzte Richtungen, wie durch die Vektorsymbole 44, 46 angedeutet ist. Aufgrund der Flexibilität der Spulenanordnung 36 kann diese aber auch in eine andere Gestalt gebracht werden. Dies ist im Zusammenhang mit Figur 1 dargestellt, wo auch ein Bein mit einem Wundbereich 10 als Beispiel eines erkrankten Körperbereichs gezeigt ist. Nun liegen sich die Flächen 40, 42 gegenüber und die in den jeweiligen Segmenten der Spule erzeugten Magnetfelder haben die gleiche Richtung. Dies ist in Figur 7, in der auch ein mit der Spulenanordnung gekoppelter Funktionsstromgenerator 62 gezeigt ist, nochmals veranschaulicht, nämlich durch den einheitlichen Magnetfeldvektor 44, 46, der beide Flächen 40, 42 durchdringt. Gemäß Figur 1 durchdringt das Magnetfeld den erkrankten Körperbereich im Wesentlichen senkrecht zur Längsachse der Extremität.

Ebenfalls ist es möglich, dass die Extremität die von den Schlaufen der achtförmigen Spule definierten Flächen durchdringt, so dass dann das Magnetfeld im Wesentlichen parallel zur Achse der Extremität verläuft.

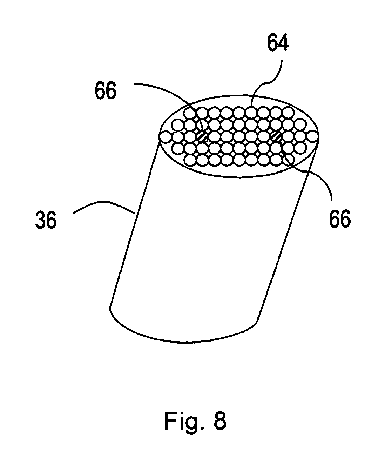

Figur 8 zeigt eine geschnittene perspektivische Teildarstellung einer Spulenanordnung mit plastischen Eigenschaften. Innerhalb der Spulenanordnung 36 ist die elektrisch leitende Spulenwicklung 64 zu erkennen. Zusätzlich sind parallel zu der Spulenwicklung 64 zwei Adern 66 aus plastischem Material vorgesehen, die der Spulenanordnung 36 insgesamt eine plastische Flexibilität vermitteln.

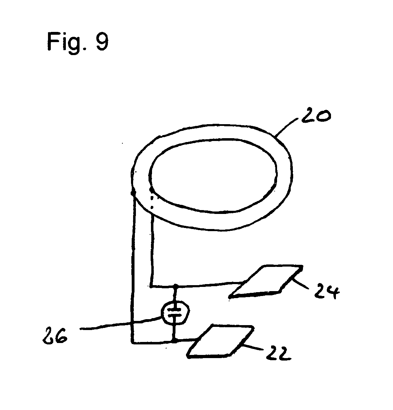

Figur 9 zeigt eine Anordnung, die im Rahmen einer erfindungsgemäßen Vorrichtung einsetzbar ist. Es ist eine Übertragerspule 20 dargestellt, deren Pole jeweils mit einer Wundelektrode 22, 24 verbunden sind. Diese Anordnung kann zusätzlich zu der im Zusammenhang mit der in Figuren 1 bis 7 erläuterten Vorrichtung vorgesehen sein. Durch das den Wundbereich direkt durchflutende elektromagnetische Feld wird auch die Übertragerspule 20, die die Rolle einer Sekundärspule einnimmt, erregt, so dass zwischen den Wundelektroden 22, 24 eine elektrische Spannung auftritt. Diese Wundelektroden 22, 24 können gezielt im Wundbereich angeordnet werden und so den Heilprozess in nützlicher Weise unterstützen. Um Spannungen zu vermeiden, die über das gewebeverträgliche Maß hinausgehen, ist eine Überspannungsschutzdiode 26 zur Spannungsbegrenzung vorgesehen. Auf der Grundlage dieser Anordnung gelingt die Kontrolle der bakteriellen Infektion einer Wunde und ihrer antibiotischen Behandlung. Mit den zur Stimulation der Wundheilung vorgesehenen Proportionen der EMF-Induktion können sowohl die Aktivität der Antibiotika-Behandlung um ca. 80 % erhöht als auch deren hemmende Wirkung auf die Wundheilung vermieden werden. Bei Infektionen chronischer Wunden bspw. mit MRSA (Methicillin-resistenter Staphylococcus aureus), einem der gefährlichsten Keime, erweist sich die magnetisch induzierte Elektrostimulation unter pH-Kontrolle und einer zusätzlichen Anwendung von MetallElektroden im (galvanischen) Kontakt mit der Wundoberfläche als unerlässlich. Bei invitro-Versuchen mit Elektroden in der Bakterien-Suspension konnte damit die Wirkung des Antibiotikum Gentamicin bei Staphylococcus aureus auf 98 % gesteigert werden. Im Hinblick auf die zunehmende Infektionsgefahr, vor allem in der chirurgischen Klinik ist diese Entdeckung von einem hohen sozialen und volkswirtschaftlichen Wert. Es ist auch möglich, die von der Übertragerspule 20 erzeugte Wechselspannung gleichzurichten oder ihr eine teilweise Gleichspannungscharakteristik zu vermitteln. Dann sollte die als Anode wirkende Wundelektrode außerhalb der Wunde, insbesondere auf der gesunden Haut, und die als Kathode wirkende Wundelektrode auf der Wunde angeordnet werden.

Die in der vorstehenden Beschreibung, in den Zeichnungen sowie in den Ansprüchen offenbarten Merkmale der Erfindung können sowohl einzeln als auch in beliebiger Kombination für die Verwirklichung der Erfindung wesentlich sein.

### Bezugszeichenliste:

- 10: Erkrankter Körperbereich
- 12: Steuereinheit
- 14: Eingangsschnittstelle / Lesegerät / Empfänger
- 16: Sensoranordnung / pH-Sensor
- 18: Übertragungseinrichtung / RFID-Transponder / Sender
- 20: Übertragerspule
- 22: Wundelektrode
- 24: Wundelektrode
- 26: Überspannungsschutzdiode
- 30: Bein
- 36: Spulenanordnung / Magnetspule / Spule
- 38: Kreuzungspunkt
- 40: Fläche
- 42: Fläche
- 44: Magnetfeld / Magnetteldvektor / Vektorsymbol
- 46: Magnetfeld / Magnetfeldvektor / Vektorsymbol
- 48: Befestigungsmittel
- 50: Befestigungsmittel
- 60: Kopplungseinrichtung
- 62: Funktionsstromgenerator
- 64: Spulenwicklung
- 66: Plastische Ader

## Patentansprüche

1. Vorrichtung zum Stimulieren eines Heilungsprozesses mit
- einem Funktionsstromgenerator (62),
- einer mit dem Funktionsstromgenerator (62) gekoppelten Spulenanordnung (36) zum Erzeugen eines elektromagnetischen Feldes in einem erkrankten Körperbereich (10) und
- einer Steuereinheit (12) zum Beeinflussen eines von dem Funktionsstromgenerator (62) erzeugten Spannungsverlaufs in Abhängigkeit von an eine Eingangsschnittstelle (14) der Steuereinheit übertragenen Signalen,
**dadurch gekennzeichnet,**
- **dass** eine Sensoranordnung (16) zum Erfassen einer Eigenschaft des erkrankten Körperbereichs (10) vorgesehen ist,
- **dass** eine mit der Sensoranordnung (16) gekoppelte Übertragungseinrichtung (18) zum Übertragen von für die erfasste Eigenschaft des erkrankten Körperbereichs charakteristischen Signalen an die Steuereinheit vorgesehen ist und
- **dass** der Funktionsstromgenerator (62) geeignet ist, in Abhängigkeit von an die Eingangsschnittstelle (14) der Steuereinheit (12) von der Sensoranordnung (16) übertragenen Signalen quasi rein-harmonische Spannungsverläufe mit einem ersten Oberwellenanteil oder entartet-harmonische Spannungsverläufe mit einem zweiten Oberwellenanteil, der größer als der erste Oberwellenanteil ist, zu erzeugen.

2. Vorrichtung nach Anspruch 1, **dadurch gekennzeichnet, dass** die Sensoranordnung einen pH-Sensor (16) umfasst.

3. Vorrichtung nach Anspruch 1 oder 2, **dadurch gekennzeichnet, dass** die Übertragungseinrichtung mindestens eine elektrische Leitung umfasst, die direkt mit der Eingangsschnittstelle der Steuereinheit in Verbindung steht.

4. Vorrichtung nach einem der vorangehenden Ansprüche, **dadurch gekennzeichnet, dass** die Übertragungseinrichtung mindestens einen Sender (18) zur drahtlosen Kommunikation mit einem der Eingangsschnittstelle der Steuereinheit (12) zugehörigen Empfänger (14) aufweist.

5. Vorrichtung nach einem der vorangehenden Ansprüche, **dadurch gekennzeichnet, dass** die Übertragungseinrichtung mindestens einen RFID-Transponder (18) aufweist, dessen Informationsgehalt von einem der Eingangsschnittstelle der Steuereinheit (12) zugehörigen Lesegerät (14) erfassbar ist.

6. Vorrichtung nach Anspruch 5, **dadurch gekennzeichnet, dass** der auslesbare Informationsgehalt des RFID-Transponders (18) in Abhängigkeit von Signalen, die von der Sensoranordnung (16) geliefert werden, veränderbar ist.

7. Vorrichtung nach Anspruch 5 oder 6, **dadurch gekennzeichnet, dass** mehrere RFID-Transponder vorgesehen sind, die in Abhängigkeit von Signalen, die von der Sensoranordnung geliefert werden, aktivierbar oder deaktivierbar sind.

8. Vorrichtung nach einem der vorangehenden Ansprüche, **dadurch gekennzeichnet, dass** die Sensoranordnung (16) einen ionensensitiven Feldeffektransistor aufweist.

9. Vorrichtung nach einem der vorangehenden Ansprüche, **dadurch gekennzeichnet, dass** die Spulenanordnung eine Spule mit einer einen Kreuzungspunkt (38) aufweisenden Spulenwicklung aufweist, die durch eine achtförmige Gestalt zwei Flächen definiert (40, 42), wobei die Flächen (40, 42) so zueinander ausgerichtet sind, dass die von einem durch einen Stromfluss in der Spulenanordnung (36) erzeugten, die Flächen durchdringenden Magnetfelder (44, 46) im Wesentlichen gleichgerichtet sind.

10. Vorrichtung nach Anspruch 9, **dadurch gekennzeichnet, dass** die Spulenanordnung (36) flexibel ist, so dass die Flächen (40, 42) auf gegenüberliegenden Seiten des erkrankten Körperbereichs (10) anordenbar sind.

11. Vorrichtung nach einem der vorangehenden Ansprüche, **dadurch gekennzeichnet, dass** der Funktionsstromgenerator (62) geeignet ist, bei einem von der Sensoranordnung erfassten pH-Wert unter 7 einen quasi rein-harmonischen Spannungsverlauf mit einer Frequenz zwischen 1 und 30 Hz zu erzeugen und bei einem von der Sensoranordnung erfassten pH-Wert über 7 einen entartet-harmonischen Spannungsverlauf mit einer Grundfrequenz zwischen 1 und 30 Hz und physiologisch wirksamen Oberwellen mit einem fünf- bis fünfzigfachen der Grundfrequenz zu erzeugen.

12. Vorrichtung nach einem der vorangehenden Ansprüche, enthaltend eine Übertragerspule (20) und mit dieser gekoppelte Elektroden (22, 24), welche zur Anordnung in oder an dem erkrankten Körperbereich (10) vorgesehen sind.

13. Vorrichtung nach Anspruch 12, **dadurch gekennzeichnet, dass** sie geeignet ist, eine von der Übertragerspule (20) erzeugte Wechselspannung gleichzurichten.

14. Vorrichtung nach Anspruch 12 oder 13, **dadurch gekennzeichnet, dass** sie geeignet ist, einer von der Übertragerspule (20) erzeugten Wechselspannung eine teilweise Gleichspannungscharakteristik zu vermitteln.

15. Vorrichtung nach Anspruch 13 oder 14, enthaltend eine als Anode (22) wirkende Elektrode, welche geeignet ist, außerhalb einer Wunde angeordnet zu werden, und eine als Kathode (24) wirkende Elektrode, welche geeignet ist, auf der Wunde angeordnet zu werden.

## Claims

1. A device for stimulating a healing process, comprising
- a functional current generator (62),
- a coil arrangement (36) coupled to the functional current generator (62) for generating an electromagnetic field in an affected body region (10) and
- a control unit (12) for influencing a voltage profile generated by the functional current generator (62) in dependence on signals transmitted to an input interface (14) of the control unit,
**characterized in that**
- a sensor arrangement (16) for detecting a property of the affected body region (10) is provided,
- a transmission device (18) coupled to the sensor arrangement (16) for transmitting signals characteristic of the detected property of the affected body region to a control unit is provided, and
- the functional current generator (62) is capable of generating, in dependence on signals transmitted from the sensor arrangement (16) to the input interface (14) of the control unit (12), essentially purely harmonic voltage profiles with a first harmonic wave contribution or degenerated harmonic voltage profiles with a second harmonic contribution greater than the first harmonic contribution.

2. The device of claim 1, **characterized in that** the sensor arrangement comprises a pH sensor (16).

3. The device of claim 1 or 2, **characterized in that** the transmission device comprises at least one electric line connected directly to the input interface of the control unit.

4. The device of one of the preceding claims, **characterized in that** the transmission device comprises at least one transmitter (18) for wireless communication with a receiver (14) associated with the input interface of the control unit (12).

5. The device of one of the preceding claims, **characterized in that** the transmission device comprises at least one RFID transponder (18) having a content of information that is readable by a reading device (14) associated with the input interface of the control unit (12).

6. The device of claim 5, **characterized in that** the readable content of information of the RFID transponder (18) can be modified in dependence on signals delivered by the sensor arrangement (16).

7. The device of claim 5 or 6, **characterized in that** several RFID transponders are provided, which can be activated or deactivated in dependence on signals delivered by the sensor arrangement.

8. The device of one of the preceding claims, **characterized in that** the sensor arrangement (16) comprises an ion-sensitive field-effect transistor.

9. The device of one of the preceding claims, **characterized in that** the coil arrangement comprises a coil with a coil wiring having a crossing point (38) defining two surfaces (40, 42) by way of a shape in the form of an eight, the surfaces (40, 42) being oriented relative to each other such that the magnetic fields (44, 46) generated by a current flow in the coil arrangement (36) and penetrating the surfaces are oriented in substantially the same direction.

10. The device of claim 9, **characterized in that** the coil arrangement (36) is flexible, so that the surfaces (40, 42) can be arranged on opposite sides of the affected body region (10).

11. The device of one of the preceding claims, **characterized in that** the functional current generator (62) is capable of generating an essentially purely harmonic voltage profile with a frequency between 1 and 30 Hz if a pH value captured by the sensor arrangement is less than 7 and to generate a degenerated harmonic voltage profile with a fundamental frequency between 1 and 30 Hz and physiologically effective harmonics of 5 to 50 times the fundamental frequency if a pH value captured by the sensor arrangement is greater than 7.

12. The device of one of the preceding claims, containing a transmission coil (20) and electrodes (22, 24) coupled thereto, which are provided to be positioned in or next to the affected body region (10).

13. The device of claim 12, **characterized in that** it is capable of rectifying an alternating voltage generated by the transmission coil (20).

14. The device of claim 12 or 13, **characterized in that** it is capable of imposing a partial continuous voltage characteristic on an alternating voltage generated by the transmission coil (20).

15. The device of claim 13 or 14, containing an electrode acting as an anode (22), which is suitable for being positioned outside a wound, and an electrode acting as a cathode (24), which is suitable for being positioned on the wound.

## Revendications

1. Dispositif pour stimuler un processus de guérison comportant
- un générateur de courant fonctionnel (62),
- un agencement de bobine (36) couplé au générateur de fonctionnel (62) pour générer un champ électromagnétique dans une partie atteinte (10) du corps et
- une unité de commande (12) pour influencer un tracé de tension généré par le générateur de courant fonctionnel (62) en fonction de signaux transmis à une interface d'entrée (14) de l'unité de commande,
**caractérisé en ce**
- **qu'**un agencement de capteur (16) pour cerner une propriété de la partie atteinte du corps (10) est prévu,
- **qu'**un dispositif de transmission (18) couplé à l'agencement de capteur (16) pour transmettre des signaux caractéristiques d'une propriété cernée de la partie atteinte du corps à l'unité de commande est prévu et
- **que** le générateur de courant fonctionnel (62) est à approprié pour générer des tracés de tension quasi purement harmoniques avec une première contribution d'onde harmonique ou des tracés de tension harmoniques dégénérés avec une deuxième contribution d'onde harmonique, qui est plus grande que la première contribution d'onde harmonique, en fonction de signaux transmis de l'agencement de capteur (16) à l'interface d'entrée (14) de l'unité de commande (12).

2. Dispositif selon la revendication 1, **caractérisé en ce que** l'agencement de capteur comporte un détecteur pH (16).

3. Dispositif selon la revendication 1 ou 2, **caractérisé en ce que** le dispositif de transmission comporte au moins une ligne électrique reliée directement à l'interface d'entrée de l'unité de commande.

4. Dispositif selon l'une des revendications précédentes, **caractérisé en ce que** le dispositif de transmission comporte au moins un émetteur (18) pour la communication sans fil avec un récepteur (14) associé à l'interface d'entrée de l'unité de commande (12).

5. Dispositif selon l'une des revendications précédentes, **caractérisé en ce que** le dispositif de transmission comporte au moins un transpondeur RFID (18) dont le contenu informatif peut être saisi par un dispositif lecteur (14) associé à l'interface d'entrée de l'unité de commande (12).

6. Dispositif selon la revendication 5, **caractérisé en ce que** le contenu informatif lisible du transpondeur RFID (18) peut être modifié en fonction de signaux fournis par l'agencement de capteur (16).

7. Dispositif selon la revendication 5 ou 6, **caractérisé en ce que** plusieurs transpondeurs RFID sont prévus qui peuvent être activés ou désactivés en fonction de signaux fournis par l'agencement de capteur.

8. Dispositif selon l'une des revendications précédentes, **caractérisé en ce que** l'agencement de capteur (16) comporte un transistor à effet de champ qui est sensitif aux ions.

9. Dispositif selon l'une des revendications précédentes, **caractérisé en ce que** l'agencement de bobine comporte une bobine avec un bobinage comportant un point de croisement (38) qui définit, par une forme de huit, deux surfaces (40, 42), les surfaces (40, 42) étant orientées l'une par rapport à l'autre de sorte que les champs magnétiques (44, 46) générés par un flux de courant dans l'agencement de bobine (36) et pénétrant les surfaces sont orientés essentiellement dans la même direction.

10. Dispositif selon la revendication 9, **caractérisé en ce que** l'agencement de bobine (36) est pliable de sorte que les surfaces (40, 42) peuvent être positionnées sur des côtés opposés de la partie atteinte (10) du corps.

11. Dispositif selon l'une des revendications précédentes, **caractérisé en ce que** le générateur de courant fonctionnel (62) est approprié pour générer un tracé de tension quasi purement harmonique avec une fréquence entre 1 et 30 Hz lorsqu'une valeur pH saisie par l'agencement de capteur est inférieure à 7 et de générer un tracé de tension harmonique dégénéré avec une fréquence fondamentale entre 1 et 30 Hz et des ondes harmoniques à effet physiologique avec un multiple de la fréquence fondamentale qui vaut entre 5 et 15 lorsqu'une valeur pH saisie par l'agencement de capteur est supérieure à 7.

12. Dispositif selon l'une des revendications précédentes, contenant une bobine translative (20) et des électrodes (22, 24) couplées à celle-ci, qui sont prévues à être positionnées dans ou à coté de la partie atteinte (10) du corps.

13. Dispositif selon la revendication 12, **caractérisé en ce qu'**il est approprié pour redresser une tension alternative générée par la bobine translative (20).

14. Dispositif selon la revendication 12 ou 13, **caractérisé en ce qu'**il est approprié pour conférer une caractéristique partielle de tension continue à une tension alternative générée par la bobine translative (20).

15. Dispositif selon la revendication 13 ou 14, contenant une électrode agissant comme une anode (22) qui est appropriée à être positionnée hors de la plaie et une électrode agissant comme une cathode (24) qui est appropriée à être positionnée sur la plaie.
